# EUROPÄISCHE PATENTSCHRIFT

(11) **EP 2 802 303 B1**
(45) Veröffentlichungstag und Bekanntmachung des Hinweises auf die Patenterteilung: **04.04.2018**
(21) Anmeldenummer: 14706773.0
(22) Anmeldetag: 20.01.2014
(51) Int. Cl.: A61F 9/007

(54) **VORRICHTUNG ZUM SCHNEIDEN UND ABSAUGEN VON GEWEBE**
DEVICE FOR CUTTING AND ASPIRATING TISSUE
DISPOSITIF DE COUPE ET D'ASPIRATION DE TISSU

(30) Priorität: 04.02.2013 DE 102013201784
(43) Veröffentlichungstag der Anmeldung: 19.11.2014
(73) Patentinhaber: Geuder AG, 69126 Heidelberg (DE)
(72) Erfinder: RIEGER, Frank, 69254 Malsch (DE); DRAHEIM, René, 69207 Sandhausen (DE); FRAUENFELD, Dieter, 69121 Heidelberg (DE); GEUDER, Volker, 69126 Heidelberg (DE)
(74) Vertreter: Patent- und Rechtsanwälte Ullrich & Naumann
(86) Internationale Anmeldenummer: PCT/DE2014/200007
(87) Internationale Veröffentlichungsnummer: WO 2014/117774

(56) Entgegenhaltungen:
- WO-A1-94/09711
- CH-A2- 704 384
- DE-A1-102010 050 337
- US-A- 4 111 207
- US-A- 4 696 298
- US-B1- 6 258 111

## Beschreibung

Die Erfindung betrifft eine Vorrichtung zum Schneiden und Absaugen von Gewebe aus dem menschlichen oder tierischen Körper, insbesondere zum Einsatz in der Vitrektomie, zum Netzhautpeeling, etc., mit einer äußeren Röhre und einer in der äußeren Röhre konzentrisch, mit geringem Spiel alternierend verschiebbaren inneren Röhre, wobei die äußere Röhre am freien Ende geschlossen ist und nahe dem freien Ende mindestens eine seitliche Öffnung mit mindestens einer inneren Schneidkante aufweist, wobei die innere Röhre am freien Ende offen ist und dort eine äußere Schneidkante aufweist und wobei die Schneidkanten beim Verschieben der inneren Röhre gegenüber der äußeren Röhre schneidend zusammenwirken.
Grundsätzlich geht es hier um ein chirurgisches Schneidinstrument zur Entnahme von Gewebe. Mit dem Instrument lässt sich das Gewebe - am bzw. im Körper - schneiden und vom Körper bzw. aus dem Körper heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen. Eine Vorrichtung der gattungsgemäßen Art eignet sich grundsätzlich zum Einsatz in der Augenchirurgie.
Zum Stand der Technik sei lediglich beispielhaft auf die DE 10 2010 050 337 A1 verwiesen. Aus dieser Druckschrift ist eine gattungsgemäße Vorrichtung bekannt, wobei dort sowohl die äußere Röhre als auch die innere Röhre jeweils zwei seitliche Ausnehmungen mit Doppelschneidfunktion hat. Ähnliche Vorrichtungen sind aus der US 5,474,532, US 6,258,111 und US 5,106,364 bekannt. Für die gattungsbildenden Vorrichtungen ist typisch, dass zwischen der Innenwandung der äußeren Röhre und der Außenwandung der inneren Röhre ein äußerst geringes Spiel realisiert sein muss, so dass die jeweiligen Schneidkanten in idealer Weise zusammenwirken. Das Gewebe soll durch Zusammenwirken der Schneidkanten geschnitten und nicht etwa gequetscht oder abgeschert werden. Ein ideales Zusammenwirken der Schneidkanten bei geringstmöglichem Spiel ist angestrebt.

Bei der hier in Rede stehenden Vorrichtung, insbesondere zur Anwendung in der Augenchirurgie, sind kleinste Abmessungen zu realisieren. So gibt es Vorrichtungen der gattungsbildenden Art, bei denen die innere Röhre eine Wandstärke von vier Hundertstel Millimeter hat. Die äußere Röhre hat einen Außendurchmesser im Bereich von 0,5 bis 0,9 mm und einen Innendurchmesser von 0,35 bis 0,37 mm. Entsprechend ist der Außendurchmesser der inneren Röhre, mit geringstmöglichem Spiel, an den Innendurchmesser der äußeren Röhre anzupassen. Dies ist in der Konstruktion aufwändig und verursacht daher erhebliche Kosten bei der Fertigung, jedenfalls dann, wenn ein hinreichend gutes Schneidergebnis erzielt werden soll.

Der vorliegenden Erfindung liegt die Aufgabe zugrunde, die gattungsbildende Vorrichtung derart auszugestalten und weiterzubilden, dass auch bei größeren Toleranzen ein hinreichend gutes Schneidergebnis realisiert wird, wobei die Vorrichtung einfach in der Konstruktion und günstig zu fertigen sein soll.

Voranstehende Aufgabe ist durch die Merkmale des Patentanspruchs 1 gelöst. Danach ist die gattungsbildende Vorrichtung gekennzeichnet durch eine formgebende Maßnahme in bzw. an der äußeren Röhre und/oder in bzw. an der inneren Röhre, wonach die innere Röhre zumindest im Bereich der seitlichen Öffnung der äußeren Röhre unter zumindest geringer Vorspannung in der äußeren Röhre läuft.

Der Erfindung liegt die Idee zugrunde, dass die in der äußeren Röhre laufende innere Röhre gegenüber der Innenwandung der äußeren Röhre vorgespannt ist, ähnlich wie dies bei den Klingen einer Schere der Fall ist. Eine solche Vorspannung hat den enormen Vorteil, dass ein gutes Schneidergebnis auch bei größeren Toleranzen zwischen dem Außendurchmesser der inneren Röhre und dem inneren Durchmesser der äußeren Röhre realisierbar ist, nämlich dann, wenn die Vorspannung zumindest im Bereich der zusammenwirkenden Schneidkanten realisiert ist. Dabei ist sicherzustellen, dass bspw. die vordere Schneidkante der inneren Röhre nicht in die seitliche Ausnehmung der äußeren Röhre greift, vielmehr die Schneidkanten unter Realisierung der zumindest geringen Vorspannung aneinander schneidend vorbeigleiten. Durch die bogenförmige Ausgestaltung der Schneidkanten findet auch tatsächlich eine Schneidbewegung und nicht etwa ein Abquetschen oder Abscheren des Gewebes statt.

Die in erfindungsgemäßer Weise vorgesehene formgebende Maßnahme lässt sich durch unterschiedliche Ausprägungen realisieren. Zum einen ist es möglich, die innere Röhre dort an die Innenwandung der äußeren Röhre zu drücken, wo die innere Schneidkante der äußeren Röhre ausgebildet ist. Dies kann durch eine Deformation in der Innenwandung der äußeren Röhre und/oder der Außenwandung der inneren Röhre erfolgen, möglichst auf der der Öffnung der äußeren Röhre gegenüberliegenden Seite. Ebenso ist es denkbar, dass die äußere Röhre und/oder die innere Röhre gegenüber der Längsachse abgewinkelt ist, dergestalt, dass die innere Röhre unter Vorspannung in der äußeren Röhre läuft und dabei die Schneidkanten unter Vorspannung aneinander entlang gleiten, wie dies bei einer Schere der Fall ist. Zu konkreten Ausgestaltungsmöglichkeiten der erfindungsgemäßen Vorrichtung nun Folgendes:
Im einfachsten Falle hat die äußere Röhre eine einzige seitliche Öffnung, so dass es sich dabei um einen sog. Einfachschneider handelt. Die innere Röhre kann am freien Ende, d.h. an der vorderen Öffnung, eine einzige Schneidkante aufweisen. Ebenso ist es denkbar, dass im Rahmen einer solchen Ausgestaltung auch die innere Röhre mit einer seitlichen Öffnung ausgestattet ist, wobei die Öffnung der inneren Röhre eine im Rückhub wirkende äußere Schneidkante im Bereich der Öffnung aufweist. Bei einer solchen Ausgestaltung kann die Vorrichtung beim Vorhub und beim Rückhub schneiden, nämlich aufgrund der beiden Schneidkanten der inneren Röhre.

Auch ist es denkbar, dass die äußere Röhre, nahe der ersten seitlichen Öffnung bzw. kurz dahinter, eine zweite seitliche Öffnung mit einer oder zwei inneren Schneidkanten aufweist. Durch diese Maßnahme lässt sich die Schneidleistung ganz erheblich erhöhen, insbesondere dann, wenn auch die innere Röhre, nahe dem freien offenen Ende, eine seitliche Öffnung mit einer oder zwei äußeren Schneidkanten aufweist, wie dies bereits zuvor ausgeführt worden ist. Jede Öffnung kann dabei eine doppelte Schneidfunktion, für den Vorhub und den Rückhub, aufweisen.

In vorteilhafter Weise ist zwischen dem offenen Ende und der seitlichen Öffnung der inneren Röhre ein nur kleiner bzw. dünner Steg ausgebildet, um nämlich so wenig wie möglich Überschneidungen zu haben. Ein sog. Saugschatten, der eine pulsierende Förderleistung bedingt, lässt sich dadurch nahezu insgesamt eliminieren. Eine maximale und gleichbleibende Saugleistung/Strömung in der Aspiration (Absaugung) lässt sich durch diese Maßnahme erreichen.

Zur Begünstigung der Strömung/Absaugleistung hat die seitliche Öffnung der inneren Röhre mindestens den gleichen Strömungsquerschnitt wie das offene Ende der inneren Röhre, wodurch die Vermeidung des Saugschattens abermals begünstigt ist. Auch ist es von Vorteil, wenn die Öffnungen in der inneren Röhre jeweils in etwa den gleichen Strömungsquerschnitt wie die Öffnungen in der äußeren Röhre haben, wobei es von weiterem Vorteil ist, wenn zu jeder Position der inneren Röhre gegenüber der äußeren Röhre ein identischer wirksamer Strömungsquerschnitt realisiert ist, um nämlich einen pulsierenden Unterdruck und somit eine pulsierende Absaugleistung zu vermeiden.

Wie bereits zuvor ausgeführt, ist in erfindungsgemäßer Weise eine formgebende Maßnahme realisiert, wonach zwischen der inneren Röhre und der äußeren Röhre eine zumindest geringe Vorspannung herrscht, damit nämlich die Schneidkanten ähnlich den Schneidkanten einer Schere zusammenwirken, wodurch das Schneidergebnis begünstigt ist.

Eine solche Maßnahme kann darin gesehen werden, dass die Innenwandung der äußeren Röhre, in etwa der seitlichen Öffnung der äußeren Röhre gegenüberliegend, nach innen erhaben ausgestaltet ist, wodurch sich an der Innenwandung der äußeren Röhre eine Art Führungsbereich ergibt. Dieser Führungsbereich liegt entweder der Öffnung der äußeren Röhre unmittelbar gegenüber oder ist, ebenfalls gegenüber liegend davor angeordnet, wobei die innere Röhre auf dem Führungsbereich läuft und dadurch die Schneidkanten beim gegenseitigen Passieren zumindest geringfügig gegeneinander gedrückt werden. Der an der Innenwandung der äußeren Röhre vorgesehene erhabene Führungsbereich kann als Verformung der Wandung der äußeren Röhre ausgeführt sein, bspw. als Einkerbung, Feder, Dimpel oder aber auch als innenseitiger Materialauftrag, bspw. in Form eines Schweißpunktes oder dergleichen. Durch diese Maßnahmen lassen sich erhebliche Toleranzen ausgleichen, wobei in erfindungsgemäßer Weise erkannt worden ist, dass zur Realisierung eines guten Schneidergebnisses es nicht erforderlich ist, dass die innere Röhre über die gesamte Länge hinweg in der äußeren Röhre bei geringstmöglicher Toleranz geführt werden muss. Vielmehr stellt die erfindungsgemäße Lehre eine Abkehr von der bisherigen Idee eines geringstmöglichen Spiels zwischen der Innenwandung der äußeren Röhre und der Außenwandung der inneren Röhre dar.

Die vorherigen Ausführungen beziehen sich auf formgebende Maßnahmen an bzw. in der Wandung der äußeren Röhre. Ebenso ist es denkbar, dass als formgebende Maßnahme die Außenwandung der inneren Röhre, vorzugsweise in einem der seitlichen Öffnung der äußeren Röhre gegenüberliegenden Bereich, nach außen erhaben ausgestaltet ist, so dass der Führungsbereich der Außenwandung der inneren Röhre zugeordnet ist. Grundsätzlich gelten hier die gleichen Ausführungen wie zu den zuvor genannten Maßnahmen an bzw. in der Wandung der äußeren Röhre.

Wie bereits zuvor ausgeführt, lässt sich der Führungsbereich bei der äußeren Röhre aus einer von außen nach innen gerichteten Verformung und/oder bei der inneren Röhre aus einer von innen nach außen gerichteten Verformung oder aber durch einen entsprechenden Materialauftrag entweder auf der Innenwandung der äußeren Röhre oder auf der Außenwandung der inneren Röhre realisieren. Ebenso ist eine Kombination beider Maßnahmen - versetzt zueinander und sowohl an der äußeren Röhre als auch an der inneren Röhre - denkbar.

Alternativ oder ergänzend lässt sich die zumindest geringe Vorspannung zwischen der inneren Röhre und der äußeren Röhre, zumindest im Bereich der Schneidkanten, dadurch realisieren, dass als formgebende Maßnahme die innere Röhre und/oder die äußere Röhre, vor der Öffnung bzw. den Öffnungen und somit vor den Schneidkanten, derart gebogen bzw. gegenüber der Längsachse abgewinkelt ist/sind, dass beim alternierenden Verschieben der inneren Röhre in der äußeren Röhre die Schneiden unter zumindest geringer Vorspannung einander passieren. Durch die Abwinkelung, bspw. der inneren Röhre, lässt sich die innere Röhre derart in die äußere Röhre einbauen, dass die Schneidkante der inneren Röhre zumindest geringfügig gegen die Innenwandung der äußeren Röhre gedrückt wird, so dass beim Passieren der Öffnung in der äußeren Röhre die Schneidkanten unter zumindest geringer Vorspannung ähnlich den Schneidkanten von Scherenblättern aneinander entlang gleiten und in idealer Weise schneiden.

Zur Vermeidung einer Verkantung bzw. zur Begünstigung eines einwandfreien Bewegungsablaufs der inneren Röhre innerhalb der äußeren Röhre ist es denkbar und von weiterem Vorteil, wenn die innere Röhre zwei gegensinnige Abwinkelungen aufweist, nämlich zum einen eine Abwinkelung zur grundsätzlichen Erzeugung der Vorspannung und zum anderen eine entgegengesetzte Abwinkelung, möglichst nahe dem distalen Bereich, so dass der Eingriffswinkel der Schneidkante der inneren Röhre in die Öffnungen der äußeren Röhre die Bewegung nicht behindert, vielmehr zumindest im vorderen Bereich die Außenwandung der inneren Röhre und die Innenwandung der äußeren Röhre nahezu parallel zueinander ausgerichtet sind, jedoch aufgrund der ersten Abwinkelung unter Vorspannung.

Im Konkreten kann die vom freien Ende entfernte Abwinkelung 1° bis 2° und die dem freien Ende nahe Abwinkelung 2° bis 5° betragen. Bei üblicher Dimensionierung der erfindungsgemäßen Vorrichtung, zum Einsatz in der Ophthalmologie, ist die vom freien Ende entfernte Abwinkelung etwa 1 mm bis 2 mm vor dem freien Ende ausgeführt, bspw. 1,6 mm vom freien Ende entfernt. Die dem freien Ende nahe Abwinkelung ist vorzugsweise im Bereich der seitlichen Öffnung der inneren Röhre ausgeführt, vorzugsweise 0,1 mm bis 0,5 mm vor dem freien Ende, insbesondere etwa 0,3 mm vor dem freien offenen Ende der inneren Röhre. Die beiden Abwinkelungen können entsprechend den voranstehenden Ausführungen so ausgeführt sein, dass die Außenwandung der inneren Röhre nahezu parallel zur Außenwandung der inneren Röhre vor der ersten Abwinkelung verläuft, jedoch geringfügig dazu versetzt, um nämlich die Vorspannung der inneren Röhre gegen die äußere Röhre zu erzeugen, und zwar im Bereich der Öffnung der äußeren Röhre, damit nämlich die Schneidkanten wirksam gegeneinander arbeiten.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. Die Erfindung wird durch die Ansprüche definert. In der Zeichnung zeigen
- Fig. 1: in einer schematischen Ansicht ein Ausführungsbeispiel einer erfindungsgemäßen Vorrichtung zum Schneiden und Absaugen von Gewebe aus dem menschlichen Körper, wobei es sich dabei im Konkreten um eine Vorrichtung zum Einsatz in der Vitrektomie handelt,
- Fig. 2: in einer schematischen Seitenansicht, geschnitten, die äußere Röhre des Gegenstands aus Fig. 1 im Rahmen einer ersten Variante,
- Fig. 3: in einer schematischen Seitenansicht, geschnitten, die äußere Röhre des Gegenstands aus Fig. 1 im Rahmen einer zweiten Variante,
- Fig. 4: in einer schematischen Seitenansicht, geschnitten, die äußere Röhre des Gegenstands aus Fig. 1 im Rahmen einer dritten Variante,
- Fig. 5: in einer schematischen Seitenansicht, geschnitten, die äußere Röhre des Gegenstands aus Fig. 1 im Rahmen einer vierten Variante,
- Fig. 6: in einer schematischen Seitenansicht, geschnitten, ein weiteres Ausführungsbeispiel einer erfindungsgemäßen Vorrichtung, wonach eine Vorspannung zwischen der inneren Röhre und der äußeren Röhre durch eine doppelt abgewinkelte innere Röhre erzeugt wird und
- Fig. 7: in einer schematischen Seitenansicht, geschnitten, die doppelt abgewinkelte innere Röhre des Gegenstands aus Fig. 6.

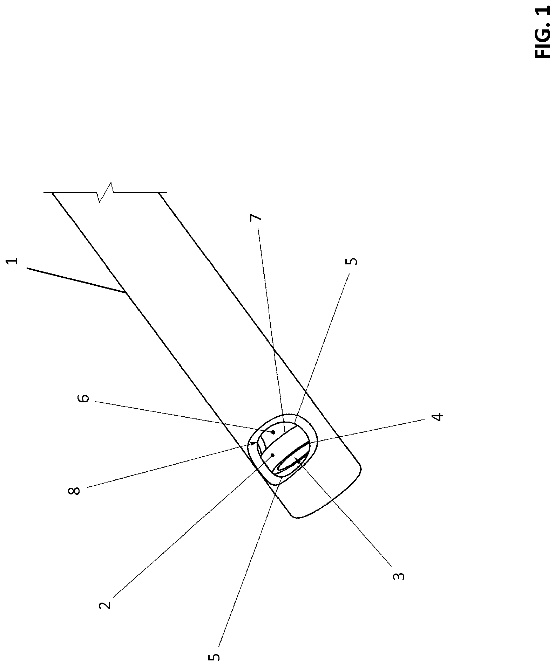

Fig. 1 zeigt in einer schematischen Ansicht, teilweise, eine erfindungsgemäße Vorrichtung zum Schneiden und Absaugen von Gewebe aus dem Auge, insbesondere zum Einsatz in der Vitrektomie.

Die Vorrichtung umfasst eine äußere Röhre 1 und eine in der äußeren Röhre 1 laufende innere Röhre 2. Die innere Röhre 2 ist in der äußeren Röhre 1 alternierend verschiebbar. Fig. 1 lässt des Weiteren erkennen, dass die äußere Röhre 1 am distalen Ende geschlossen ist. Die innere Röhre 2 hat dagegen am distalen Ende eine Öffnung 3, die mit einer äußeren Schneidkante 4 ausgestattet ist. Diese äußere Schneidkante 4 wirkt mit einer inneren Schneidkante 5 der äußeren Röhre 1 zusammen, nämlich beim Verschieben der inneren Röhre 2 gegenüber der äußeren Röhre 1.

Fig. 1 lässt des Weiteren erkennen, dass die innere Röhre 2 mit einer seitlichen Öffnung 6 ausgestattet ist, die eine im Rückhub wirkende äußere Schneidkante 7 aufweist. Beide äußeren Schneidkanten 4, 7 der inneren Röhre 2 wirken mit der inneren Schneidkante 5 der seitlichen Öffnung 8 der äußeren Röhre 1 zusammen.

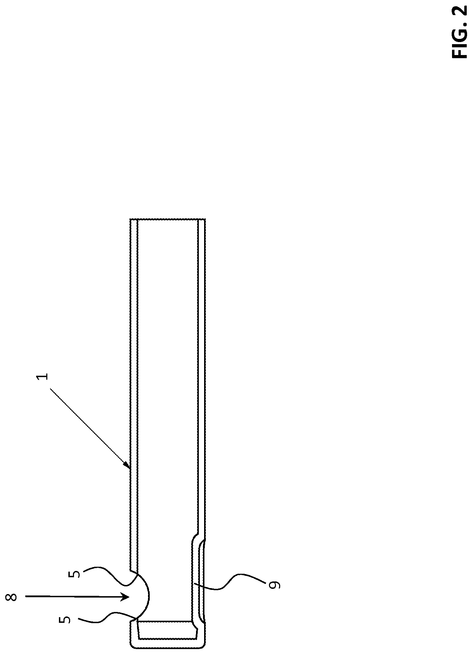

Fig. 2 zeigt in einer schematischen Seitenansicht, geschnitten, eine prinzipielle Ausgestaltung der äußeren Röhre 1, wonach diese auf der der seitlichen Öffnung 8 gegenüberliegenden Seite verformt ist, um nämlich die in Fig. 2 nicht gezeigte innere Röhre 2 mit dessen Schneidkante 4 gegen die innere Schneidkante 5 der äußeren Röhre 1 zu drücken. Durch diese Maßnahme lässt sich die erfindungsgemäße Vorspannung realisieren.

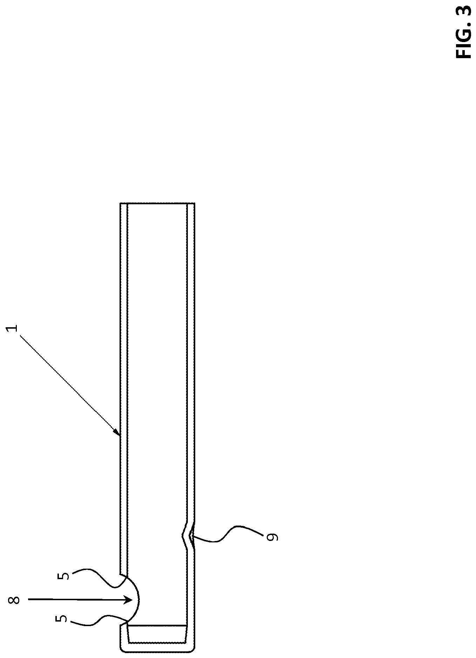

Fig. 3 zeigt eine weitere Variante der erfindungsgemäßen Lehre, realisiert durch eine Maßnahme an bzw. in der äußeren Röhre 1, ähnlich der Darstellung in Fig. 2. Im Konkreten ist in der Wandung der äußeren Röhre 1 eine Sike bzw. Prägung 9, Einkerbung oder dgl. vorgesehen, so dass auch durch diese Maßnahme die innere Röhre 2 mit ihrer äußeren Schneidkante 4 bzw. ihren Schneidkanten 4, 7 gegen die innere Schneidkante 5 der äußeren Röhre 1 gedrückt wirkt, um nämlich das Schneidergebnis auch bei erheblichem Spiel zwischen der Innenwandung der äußeren Röhre 1 und der Außenwandung der inneren Röhre 2 zu begünstigen.

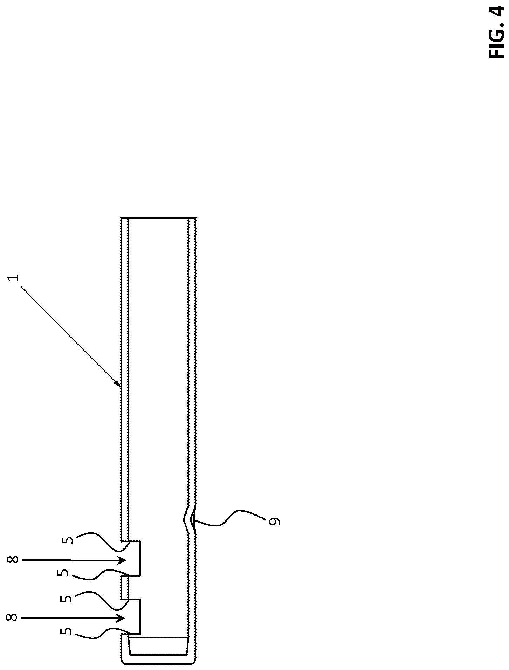

Gemäß Fig. 4 ist die äußere Röhre 1 mit zwei seitlichen Öffnungen 8 ausgestattet. Entsprechend sind mehrere Schneidkanten 5 vorgesehen, bei dem in Fig. 4 gezeigten Ausführungsbeispiel maximal vier Schneidkanten 5, zwei für den Vorhub und zwei für den Rückhub. Eine Prägung 9 sorgt für die Vorspannung bei eingeschobener innerer Röhre 2, wie dies in Fig. 1 gezeigt ist.

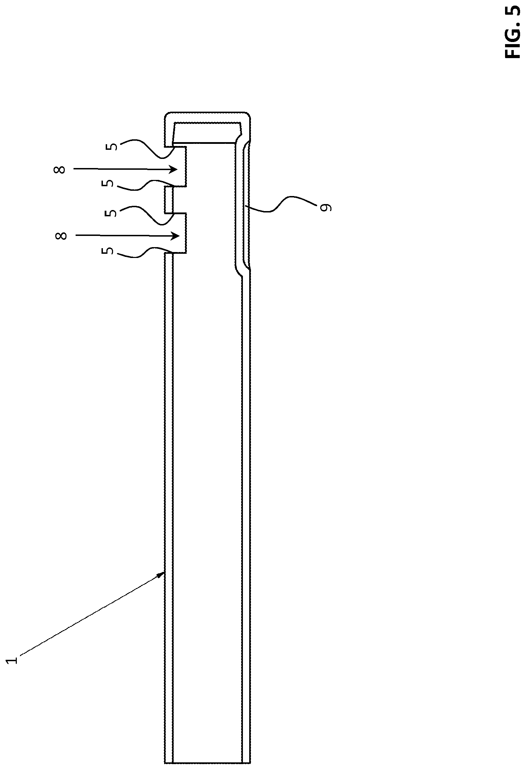

Die Darstellung in Fig. 5 ist gegenüber den Darstellungen in den Fig. 2 bis 4 spiegelverkehrt, wobei gegenüber der Darstellung in Fig. 4 eine flächige Prägung 9 vorgesehen ist, um nämlich die Vorspannung über die gesamte Strecke der beiden Öffnungen 8 hinweg zu gewährleisten.

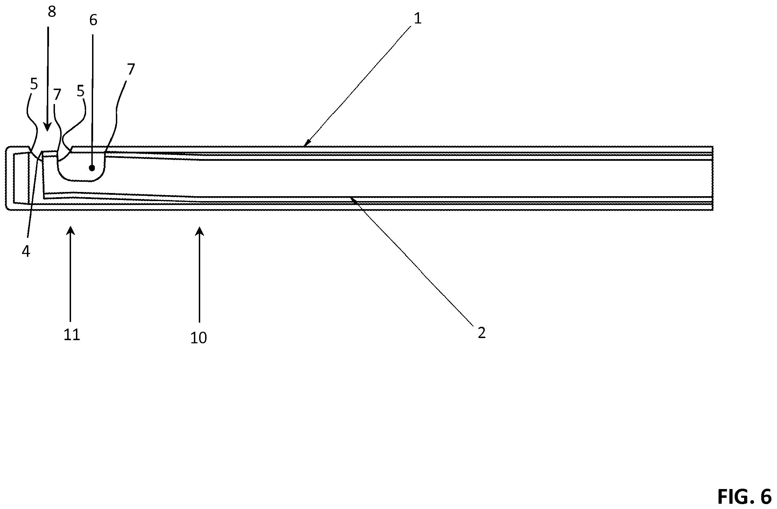

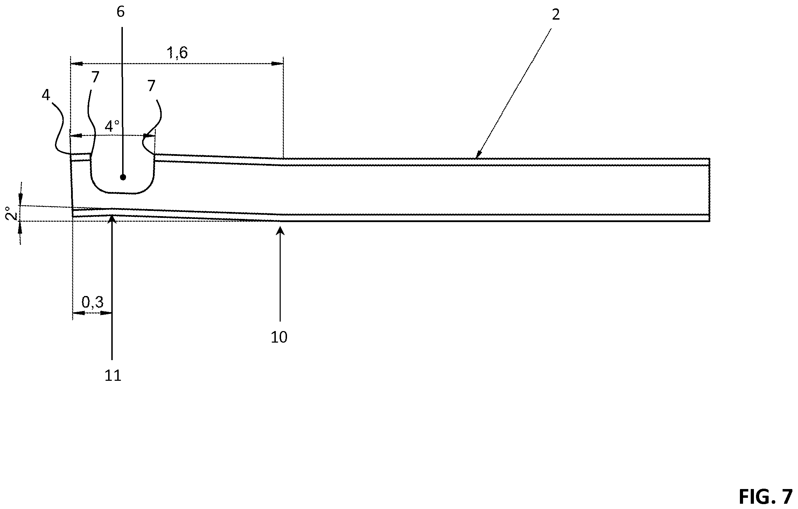

Fig. 6 zeigt eine weitere Maßnahme zur Realisierung der erfindungsgemäßen Vorspannung zwischen der äußeren Röhre 1 und der darin laufenden inneren Röhre 2. Die Vorspannung wird - alternativ oder ergänzend - zu den Maßnahmen entsprechend den Fig. 2 bis 5, durch eine doppelte Abwinkelung der inneren Röhre 2 gegenüber der Längsachse realisiert, wobei die beiden Abwinkelungen der Einzeldarstellung der inneren Röhre 2 gemäß Fig. 7 entnehmbar sind.

Eine erste Abwinkelung 10 ist bei dem in den Fig. 6 und 7 gezeigten Ausführungsbeispiele bei 1,6 mm vor dem distalen Ende der inneren Röhre 2 vorgesehen, um nämlich die äußeren Schneidkanten 4, 7 gegen die Innenwandung bzw. die innere Schneidkante 5 der äußeren Röhre 1 zu drücken. Zur quasi "Entschärfung" ist eine zweite Abwinkelung 11 vorgesehen, die die innere Röhre 2 zumindest in der Flucht geringfügig zurückbiegt, damit die Außenwandung in etwa parallel zu der Außenwandung vor der ersten Abwinkelung 10 verläuft, um nämlich einen zu starken Eingriff der äußeren Schneidkanten 4, 7 im Bereich der seitlichen Öffnung 8 der äußeren Röhre 1 zu vermeiden. Die zum distalen Ende hin ausgebildete zweite Abwinkelung 11 ist bei den in den Fig. 6 und 7 gezeigten Ausführungsbeispielen etwa 0,3 mm vom distalen Ende entfernt und ist im Bereich der seitlichen Öffnung 6 der inneren Röhre 2 vorgesehen.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: äußere Röhre
- 2: innere Röhre
- 3: Öffnung am distalen Ende der inneren Röhre
- 4: äußere Schneidkante der inneren Röhre
- 5: innere Schneidkante der äußeren Röhre
- 6: seitliche Öffnung der inneren Röhre
- 7: äußere Schneidkante der inneren Röhre
- 8: seitliche Öffnung der äußeren Röhre
- 9: Prägung
- 10: erste Abwinkelung
- 11: zweite Abwinkelung

## Patentansprüche

1. Vorrichtung zum Schneiden und Absaugen von Gewebe aus dem menschlichen oder tierischen Körper, insbesondere zum Einsatz in der Vitrektomie oder zum Netzhautpeeling, mit einer äußeren Röhre (1) und einer in der äußeren Röhre (1) konzentrisch, mit geringem Spiel alternierend verschiebbaren inneren Röhre (2), wobei die äußere Röhre (1) am freien Ende geschlossen ist und nahe dem freien Ende mindestens eine seitliche Öffnung (8) mit mindestens einer inneren Schneidkante (5) aufweist, wobei die innere Röhre (2) am freien Ende offen ist und dort eine äußere Schneidkante (4) aufweist und wobei die Schneidkanten beim Verschieben der inneren Röhre (2) gegenüber der äußeren Röhre (1) schneidend zusammenwirken, mit einer formgebenden Maßnahme in bzw. an der inneren Röhre (2), wonach die innere Röhre (2) zumindest im Bereich der seitlichen Öffnung (3) unter zumindest geringer Vorspannung in der äußeren Röhre (1) läuft und wobei die innere Röhre (2) derart abgebogen oder gegenüber der Längsachse abgewinkelt ist, dass beim alternierenden Verschieben der inneren Röhre (2) in der äußeren Röhre (1) die Schneiden unter zumindest geringer Vorspannung einander passieren, **dadurch gekennzeichnet, dass** die innere Röhre (2), nahe dem freien, offenen Ende, eine seitliche Öffnung (6) mit einer oder zwei äußeren Schneidkanten (4, 7) aufweist und dass die innere Röhre (2) vor der seitlichen Öffnung (6) gebogen ist.

2. Vorrichtung nach Anspruch 1, **gekennzeichnet durch** eine formgebende Maßnahme an der äußeren Röhre (1), wonach die innere Röhre (2) zumindest im Bereich der seitlichen Öffnung (3) unter zumindest geringer Vorspannung in der äußeren Röhre (1) läuft.

3. Vorrichtung nach Anspruch 1 oder 2, **dadurch gekennzeichnet, dass** die äußere Röhre (1), nahe der ersten seitlichen Öffnung (8), eine zweite seitliche Öffnung (8) mit einer oder zwei inneren Schneidkante(n) (5) aufweist.

4. Vorrichtung nach Anspruch 3, **dadurch gekennzeichnet, dass** zwischen dem offenen Ende und der seitlichen Öffnung (6) der inneren Röhre (2) ein nur kleiner/dünner Steg ausgebildet ist.

5. Vorrichtung nach Anspruch 3 oder 4, **dadurch gekennzeichnet, dass** die seitliche Öffnung (6) der inneren Röhre (2) mindestens den gleichen Strömungsquerschnitt wie das offene Ende der inneren Röhre (2) hat.

6. Vorrichtung nach einem der Ansprüche 1 bis 5, **dadurch gekennzeichnet, dass** die Öffnungen (6) in der inneren Röhre (2) jeweils in etwa den gleichen Strömungsquerschnitt wie die Öffnungen (8) in der äußeren Röhre (1) haben.

7. Vorrichtung nach einem der Ansprüche 1 bis 6, **dadurch gekennzeichnet, dass** als formgebende Maßnahme die Innenwandung der äußeren Röhre (1), in etwa der seitlichen Öffnung (8) gegenüberliegend, nach innen erhaben ausgestaltet ist, wodurch sich ein Führungsbereich ergibt.

8. Vorrichtung nach einem der Ansprüche 1 bis 7, **dadurch gekennzeichnet, dass** als formgebende Maßnahme die Außenwandung der inneren Röhre (2), vorzugsweise in einem der seitlichen Öffnung (6) gegenüberliegenden Bereich, nach außen erhaben ausgestaltet ist, wodurch sich ein Führungsbereich ergibt.

9. Vorrichtung nach Anspruch 7 oder 8, **dadurch gekennzeichnet, dass** der Führungsbereich bei der äußeren Röhre (1) aus einer von außen nach innen gerichteten Verformung und/oder bei der inneren Röhre (2) aus einer von innen nach außen gerichteten Verformung resultiert.

10. Vorrichtung nach Anspruch 7 oder 8, **dadurch gekennzeichnet, dass** der Führungsbereich bei der äußeren Röhre (1) als innenseitiger Materialauftrag und/oder bei der inneren Röhre (2) als außenseitiger Materialauftrag ausgeführt ist.

11. Vorrichtung nach einem der Ansprüche 1 bis 10, **dadurch gekennzeichnet, dass** als formgebende Maßnahme die äußere Röhre (1), vor der Öffnung, (8), derart gebogen oder gegenüber der Längsachse abgewinkelt ist, dass beim alternierenden Verschieben der inneren Röhre (2) in der äußeren Röhre (1) die Schneiden unter zumindest geringer Vorspannung einander passieren.

12. Vorrichtung nach Anspruch 11, **dadurch gekennzeichnet, dass** die innere Röhre (2) zwei gleichsinnige oder gegensinnige Abwinkelungen (10, 11) aufweist.

13. Vorrichtung nach Anspruch 12, **dadurch gekennzeichnet, dass** die vom freien Ende entfernte Abwinkelung (10) 1° bis 2° und die dem freien Ende nahe Abwinkelung (11) 2° bis 5° beträgt.

14. Vorrichtung nach Anspruch 12 oder 13, **dadurch gekennzeichnet, dass** die vom freien Ende entfernte Abwinkelung (10) 1 mm bis 2 mm vor dem freien Ende und die dem freien Ende nahe Abwinkelung (11) im Bereich der seitlichen Öffnung (6) der inneren Röhre (2), vorzugsweise 0,1 mm bis 0,5 mm vor dem freien Ende, liegt.

## Claims

1. Device for cutting and drawing off tissue from the human or animal body, in particular for use in vitrectomy or for retina peeling, having an outer tube (1) and an inner tube (2) which can be displaced in an alternating manner in the outer tube (1) concentrically with little play, wherein the outer tube (1) is closed at the free end and has close to the free end at least one lateral opening (8) having at least one inner cutting edge (5), wherein the inner tube (2) is open at the free end and at that location has an outer cutting edge (4) and wherein the cutting edges when the inner tube (2) is displaced relative to the outer tube (1) cooperate in a cutting manner, having a shaping means in or on the inner tube (2) according to which the inner tube (2) extends at least in the region of the lateral opening (3) with at least slight pretensioning in the outer tube (1) and wherein the inner tube (2) is bent or angled with respect to the longitudinal axis in such a manner that in the event of alternating displacement of the inner tube (2) in the outer tube (1) the cutting edges pass each other with at least slight pretensioning,
**characterised in that** the inner tube (2) close to the free open end has a lateral opening (6) having one or two outer cutting edges (4, 7) and **in that** the inner tube (2) is bent in front of the lateral opening (6).

2. Device according to claim 1, **characterised by** a shaping means on the outer tube (1), according to which the inner tube (2) extends at least in the region of the lateral opening (3) with at least slight pretensioning in the outer tube (1).

3. Device according to claim 1 or 2, **characterised in that** the outer tube (1) close to the first lateral opening (8) has a second lateral opening (8) with one or two inner cutting edge (s) (5).

4. Device according to claim 3, **characterised in that** a web which is only small/thin is formed between the open end and the lateral opening (6) of the inner tube (2).

5. Device according to claim 3 or 4, **characterised in that** the lateral opening (6) of the inner tube (2) has at least the same flow cross-section as the open end of the inner tube (2).

6. Device according to any one of claims 1 to 5, **characterised in that** the openings (6) in the inner tube (2) each have substantially the same flow cross-section as the openings (8) in the outer tube (1).

7. Device according to any one of claims 1 to 6, **characterised in that** the inner wall of the outer tube (1), substantially opposite the lateral opening (8), is constructed as a shaping means so as to protrude inwards, whereby a guiding region is produced.

8. Device according to any one of claims 1 to 7, **characterised in that** the outer wall of the inner tube (2), preferably in a region opposite the lateral opening (6), is constructed as a shaping means so as to protrude outwards, whereby a guiding region is produced.

9. Device according to claim 7 or 8, **characterised in that** the guiding region in the case of the outer tube (1) results from a deformation which is directed from the outer side to the inner side and/or in the case of the inner tube (2) from a deformation which is directed from the inner side to the outer side.

10. Device according to claim 7 or 8, **characterised in that** the guiding region in the case of the outer tube (1) is constructed as an internal material application and/or in the case of the inner tube (2) is constructed as an external material application.

11. Device according to any one of claims 1 to 10, **characterised in that** the outer tube (1) in front of the opening (8) is bent or angled with respect to the longitudinal axis as a shaping means in such a manner that, in the event of an alternating displacement of the inner tube (2) in the outer tube (1), the cutting edges pass each other with at least slight pretensioning.

12. Device according to claim 11, **characterised in that** the inner tube (2) has two bends (10, 11) in the same or opposing directions.

13. Device according to claim 12, **characterised in that** the bend (10) remote from the free end is from 1° to 2° and the bend (11) close to the free end is from 2° to 5°.

14. Device according to claim 12 or 13, **characterised in that** the bend (10) remote from the free end is located from 1 mm to 2 mm in front of the free end and the bend (11) close to the free end is located in the region of the lateral opening (6) of the inner tube (2), preferably from 0.1 mm to 0.5 mm in front of the free end.

## Revendications

1. Dispositif de coupe et d'aspiration de tissu du corps humain ou du corps animal, en particulier destiné à être utilisé en vitrectomie ou pour un pelage de la rétine, avec un tube extérieur (1) et un tube intérieur (2) pouvant se déplacer concentriquement dans le tube extérieur (1) dans un mouvement de va-et-vient avec un faible jeu, le tube extérieur (1) étant fermé à l'extrémité libre et présentant près de l'extrémité libre au moins un orifice latéral (8) avec au moins une arête de coupe intérieure (5), le tube intérieur (2) étant ouvert à l'extrémité libre et y présentant une arête de coupe extérieure (4), et les arêtes de coupe coopérant de façon coupante lors du déplacement du tube intérieur (2) par rapport au tube extérieur (1), avec une mesure de mise en forme dans ou respectivement sur le tube intérieur (2), après quoi le tube intérieur (2) court dans le tube extérieur (1) au moins dans la zone de l'orifice latéral (3) en présence d'au moins une faible précontrainte, et le tube intérieur (2) étant courbé ou coudé par rapport à l'axe longitudinal de telle sorte que, lors du déplacement dans un mouvement de va-et-vient du tube intérieur (2) dans le tube extérieur (1), les lames passent l'une devant l'autre en présence d'au moins d'une faible précontrainte, **caractérisé en ce que** le tube intérieur (2), près de l'extrémité libre ouverte, présente un orifice latéral (6) avec une ou deux arêtes de coupe extérieures (4, 7), et **en ce que** le tube intérieur (2) est courbé avant l'orifice latéral (6).

2. Dispositif selon la revendication 1, **caractérisé par** une mesure de mise en forme sur le tube extérieur (1), après quoi le tube intérieur (2) court dans le tube extérieur (1) au moins dans la zone de l'orifice latéral (3) en présence d'au moins une faible précontrainte.

3. Dispositif selon la revendication 1 ou 2, **caractérisé en ce que** le tube extérieur (1), près du premier orifice latéral (8), présente un deuxième orifice latéral (8) avec une ou deux arête(s) de coupe intérieure(s) (5).

4. Dispositif selon la revendication 3, **caractérisé en ce que**, entre l'extrémité ouverte et l'orifice latéral (6) du tube intérieur (2), il est constitué uniquement une nervure petite/mince.

5. Dispositif selon la revendication 3 ou 4, **caractérisé en ce que** l'orifice latéral (6) du tube intérieur (2) a au moins la même section transversale d'écoulement que l'extrémité ouverte du tube intérieur (2).

6. Dispositif selon l'une des revendications 1 à 5, **caractérisé en ce que** les orifices (6) dans le tube intérieur (2) ont respectivement à peu près la même section transversale d'écoulement que les orifices (8) dans le tube extérieur (1).

7. Dispositif selon l'une des revendications 1 à 6, **caractérisé en ce que**, en tant que mesure de mise en forme, la paroi intérieure du tube extérieur (1), à peu près en face de l'orifice latéral (8), est constituée de façon saillante vers l'intérieur, ce qui donne une zone de guidage.

8. Dispositif selon l'une des revendications 1 à 7, **caractérisé en ce que**, en tant que mesure de mise en forme, la paroi extérieure du tube intérieur (2), de préférence dans une zone en face de l'orifice latéral (6), est constituée de façon saillante vers l'extérieur, ce qui donne une zone de guidage.

9. Dispositif selon la revendication 7 ou 8, **caractérisé en ce que**, pour le tube extérieur (1), la zone de guidage résulte d'une déformation dirigée de l'extérieur vers l'intérieur et/ou, pour le tube intérieur (2), elle résulte d'une déformation dirigée de l'intérieur vers l'extérieur.

10. Dispositif selon la revendication 7 ou 8, **caractérisé en ce que**, pour le tube extérieur (1), la zone de guidage est réalisée en tant qu'application de matériau côté intérieur et/ou, pour le tube intérieur (2), en tant qu'application de matériau côté extérieur.

11. Dispositif selon l'une des revendications 1 à 10, **caractérisé en ce que**, en tant que mesure de mise en forme, le tube extérieur (1) est, avant l'orifice (8), courbé ou est coudé par rapport à l'axe longitudinal de telle sorte que, lors du déplacement dans un mouvement de va-et-vient du tube intérieur (2) dans le tube extérieur (1), les lames passent l'une devant l'autre en présence d'au moins une faible précontrainte.

12. Dispositif selon la revendication 11, **caractérisé en ce que** le tube intérieur (2) présente deux coudes (10, 11) de même sens ou de sens contraire.

13. Dispositif selon la revendication 12, **caractérisé en ce que** le coude (10) éloigné de l'extrémité libre est compris entre 1° et 2°, et le coude (11) proche de l'extrémité libre est compris entre 2° et 5°.

14. Dispositif selon la revendication 12 ou 13, **caractérisé en ce que** le coude (10) éloigné de l'extrémité libre est situé entre 1 mm et 2 mm avant l'extrémité libre, et le coude (11) proche de l'extrémité libre est situé dans la zone de l'orifice latéral (6) du tube intérieur (2), de préférence entre 0,1 mm et 0,5 mm avant l'extrémité libre.
